# EUROPEAN PATENT APPLICATION

(11) **EP 1 870 036 A1**
(43) Date of publication of application: **26.12.2007**
(21) Application number: 07011975.5
(22) Date of filing: 19.06.2007
(51) Int. Cl.: A61B 5/022

(54) **Blood pressure monitor**

(30) Priority: 20.06.2006 DE 102006028251
(71) Applicant: Kaz, Incorporated, New York, NY 10019 (US)
(72) Inventor: Hollinger, Stefan, 61476 Kronberg (DE); Heubach, Klaus, 65520 Bad Camberg (DE); Janouch, Peter, 60488 Frankfurt (DE); Klöppel-Riech, Michael, 61164 Friedberg (DE)
(74) Representative: Grünecker, Kinkeldey, Stockmair & Schwanhäusser Anwaltssozietät

(57) **Abstract**

The present invention relates to a blood pressure monitor having a data processing unit for management of measured data. According to this invention, the blood pressure monitor is characterized in that multiple individualizable start switches are provided for starting the device and/or a measurement operation, whereby the start switches are linked to the data processing unit such that by operation of a particular start switch, an individualizable measured data processing and/or management can be triggered. In particular, a blood pressure monitor with a memory for storage of measured values, having multiple memory sites for individualizable storage of various sets of measured values and with selection means for selecting a particular memory site for the measured values of a particular measurement is provided. The blood pressure monitor is characterized in that multiple individualized start switches are provided for starting the device and/or a measurement operation, whereby the start switches are linked to the selection means in such a way that by operating one of the start switches, a respective memory site for the particular measurement can be selected.

## Description

The present invention relates to a blood pressure monitor¹ with a data processing unit for processing and/or managing the measured data.

Blood pressure monitors of the aforementioned type are being used to an increasing extent in the home care area to perform blood pressure monitoring at home outside of the more or less regular but rare office visits with the doctor. Large volumes of data are generated here due to the frequent measurements, in some cases several times a day, and this data should be managed appropriately.

It is known that such blood pressure monitors, which may usually be designed as devices for the wrist or forearm, may be provided with a memory in which the measured values may be stored. In most cases, the measured blood pressure values are stored together with the date and time of the measurement.

Some of the devices store the measured values here automatically without further action on the part of the user. This is not only convenient but also prevents the frequently observed tendency of a hypertensive patient to suppress measured values in the memory that do not seem relevant to him but are unwelcome simply because they are too high. This forced storage of measurement values is a disadvantage, however, in the event that several people are using one device, e.g., another occupant of the household occasionally uses the device or a visitor wants to try out the device. Therefore, there is no longer any uniformity in the data in the memory when data is stored automatically in the manner described here. The subsequent interpretation is made difficult because a corresponding person such as the physician performing the analysis is no longer able to differentiate whether a specific value comes from the patient being monitored or from a third party.

On the other hand, there have been proposals for blood pressure monitors with the option of intentionally saving the measurements which eliminate the aforementioned problem with test measurements by a third party while still achieving the disadvantages described above. To remedy this situation, blood pressure monitors that have several memory traces have already been proposed, so that multiple users are able to store their data separately. In the case of automatic storage, before performing a particular measurement, it is necessary to assign that particular measurement and the corresponding measured values to a certain memory trace, e.g., this is done by the patient himself or by the life partner of the patient or one must decide whether the measured values are to be stored at all, e.g., when a visitor performs just a test measurement. Such blood pressure monitors having a plurality of memory traces may solve the problem of having intermediate measurements by a third party falsify the measurement series of the main
¹ TN: Also known as a sphygmomanometer or sphygmometer. user, but such devices with multiple user memory options have so far been accepted only hesitatingly. The step of selecting the memory trace appears to greatly complicate operation of the device.

Against this background, the object of the present invention is to create an improved blood pressure monitor of the type defined above which will avoid the disadvantages of the state of the art and will improve upon the latter in an advantageous manner. Individual measured value storage in various memory traces should preferably be made possible without having a negative effect on operation of the device or creating room for subsequent data manipulation.

This object is achieved according to this invention by a blood pressure monitor according to Claim 1. Preferred embodiments of the invention are the subject of the dependent claims.

It is proposed that the choice of a certain measured data processing and/or management shall be made by selecting different starting switches which are provided for starting the device and/or for starting a measurement operation. According to this invention, multiple individualizable starting switches are provided for starting the device and/or a measurement operation, these switches being linked to the data processing unit in such a way that individualizable measured data processing and/or management can be triggered by operating a particular starting switch. This greatly simplifies operation of the device. The particular user need only select "his" starting switch or the starting switch desired by him. By operating the particular starting switch, an individual measured data processing and/or management may be initiated without the user having to control other data processing and/or managements steps. Depending on the particular starting switch selected, various analyses of the measurement data and/or a particular management of the corresponding measured data may be performed. This management includes simple storage of the measured data assigned to a particular user. This may optionally also include such measured data that are necessary for analysis and averaging of past measurement results or for graphic analysis or which provide information about the validity of the measurement on the basis of the measurement position detected (e.g., the position of the blood pressure monitor in relation to the heart) and information about the day and/or time of the measurement.

In a further embodiment of the invention, it is proposed that the choice of the particular memory site be linked to the start of the device and/or a particular measurement operation.

The individualized start switches are advantageously linked to the starting of the device and/or a measurement operation with selection means for selecting a particular memory site, so that by operating one of the start switches; a respective memory site for the particular measurement can be selected. The memory site for the particular measurement is thus selected by operating the start switch. By means of multiple individualized started switches, different device users may start the device individually for them and/or for an individual measurement cycle for them by operating "their" starting switch and thereby cause the measured values to be stored in "their" memory trace.

The choice of the particular memory site by operation of the particular start switch advantageously is accomplished automatically and obligatorily, i.e., no additional operation of the particular starting switch, e.g., in the form of a double click or the like is necessary. Simultaneously with starting the device and/or the particular measurement operation by operating the particular starting button, the respective memory site is also selected. Advantageously the particular memory site is selected here prior to the respective measurement, so that a subsequent influence on the measured data and their storage after the measurement is no longer possible. This ensures complete data acquisition without gaps and prevents the user from eliminating unwanted data.

In a further embodiment of the invention, a change function for changing the particular memory sites may be assigned to the start switches. If a memory site is selected after starting the device, which has been triggered by operation of a start switch, then by operating another start switch, the selected memory site can be changed for the following measurement. In this way, simple operation is ensured even when the device has inadvertently been started with the wrong start switch. In this case, the device need not be turned off and then turned on again and instead it is sufficient to depress the correct start switch after inadvertently starting the device with the wrong starting switch in order to select the correct memory site for the following measurement. Each operation of one of the starting switches selects the memory site for the following measurement.

It is also possible in this way to perform blood pressure measurements on different people one after the other and assign the data thereby obtained to the memory sites provided individually for this purpose without having to power down and restart the blood pressure measurement device again each time.

In a further embodiment of the invention, to achieve intuitive operation of the device and to prevent faulty operation by actuation of the wrong start switch, the start switches are each formed by a start button individualized by individual optical markings. Various symbols may be used as the optical markings. In a further embodiment of the invention, the different start buttons are individualized by different programs and different color markings. As an alternative, writable character fields (e.g., made of cardboard or paper) are also provided so that, for example, the user's name may be written there and assigned to the particular starting button. These characters fields can be secured in the housing next to the start buttons. In a further embodiment, user names may be entered by the user based on the allocation of buttons and displayed on a display at locations that may be assigned to the particular starting buttons.

Instead of several separate starting buttons, a soft starting button may also be used, different user identifiers being assigned to said soft button, which is individualizable accordingly. This soft starting button is connectable to the aforementioned start switches, each of which starts the device and/or a measurement operation and triggers an individual measured data management, in particular the storage of the respective measured data in different individual memory traces. Depending on the occupancy of the start button with a particular user identifier and/or a particular user mode, the soft button is linked to the respective start switch to initiate the respective measured data management when starting the device and/or a measurement operation. As this shows, the various start switches need not necessarily be designed in the form of different mechanical switches, but instead may be programmed in the form of a corresponding software circuit in the data processing unit, so that each forms a soft switch so to speak.

The occupancy of the soft start button may be assigned in fundamentally different ways. According to an advantageous embodiment of the invention, the soft start button may be linked to a selection menu, preselecting various user identifiers and/or user modes from which one or more may be chosen to thereby be assigned to the soft start button. For example, different user names may be predetermined, so that a particular user selects "his" name from the predetermined user names. Alternatively or additional, the soft start button may also be linked to an input menu by means of which the individual user identifiers and/or an individual user mode can be input. For example, letter entries may be made to assign the start button accordingly. Depending on the occupancy of the soft start button, the data processing unit selects the appropriate data processing mode and/or the particular data management desired. The data processing unit may especially select a respective memory site as a function of the selected user identifier at whose address the measured data of the following measurement are stored on an individualizable basis. By selecting the particular user name in this embodiment, automatic starting of the measurement and storage of the measured data are then accomplished.

To counteract the problems mentioned above, whereby patients have a tendency to subsequently eliminate measured values they don't like, the data processing unit may have a start lock which makes the soft start button "harder" only after a user identifier has been assigned to it.

The start switches may form "off' switches at the same time in a further embodiment of the invention so that after conclusion of a measurement operation, renewed operation of the start button will shut down the device. The start button and the off button are advantageously designed here so that the off function of the device is available through each of the start buttons and off buttons, in particular even during a measurement operation, i.e., a user can operate any of the start buttons and off buttons to turn off the device and/or to terminate the measurement operation regardless of whether the device and/or the measurement operation has been initiated with the same start buttons and off buttons.

In a further embodiment of the invention, the blood pressure monitor has a display device, preferably in the form of a display for displaying the stored measured values. Retrieval means are advantageously provided for selecting a particular set of stored measured values so that the various sets of measured values assigned to the various memory sites can be displayed individually. In this way each user can see "his" data record individually. In addition to or instead of the individualized start and retrieval buttons, writable character fields may also be provided for user allocation.

The retrieval means preferably have several individualized retrieval buttons that are links to a particular storage site and, when operated, result in display of a particular set of measured values by the display device.

To allow intuitive operation of the display device without operating errors, in an advantageous further embodiment of the invention, the retrieval buttons are provided with individualized optical markings which may advantageously be provided in the form of pictograms and/or color markings. Here again, alternatively, writable character fields may be provided for user allocation.

Preferably corresponding markings are provided for the start switches and the retrieval buttons. The start switch and the respective retrieval button, which are allocated to a shared memory site, i.e., cause the storage of the measured values in a memory site on the one hand while on the other hand triggering the display of the measured values stored in this memory site, may be individualized in particular by the same optical marking which indicates that the respective start switch and the respective retrieval button belong together with respect to data management. Alternatively or additionally, the fact that the respective start button and the respective retrieval button belong together may also be indicated by a collective arrangement of the respective switches and/or buttons on the device, in particular by arranging them side by side.

As an alternative to multiple individualized retrieval buttons, in a further embodiment of the present invention, a joint retrieval button may also be provided for all memory sites. To nevertheless allow an individual display of a particular set of desired measured values, the joint retrieval button may be linked to the individualized start buttons in such a way that by actuating the joint retrieval button and additionally actuating one of the individualized start switches, the respective memory site assigned to the particular start switch may be read out and the measured values stores therein displayed by the display device.

To facilitate navigation within a set of measured values that may be quite extensive, a navigation switch is advantageously connected to the display device, so that the display of a particular set of retrieved measured values can be controlled. In particular, by operating the navigation switch within a set of measured data is possible, e.g., to the extent that it is possible to accelerate scrolling forward and/or backward through the measured data displayed, e.g., by jumping to the next measured value, jumping to the next block of measured values, e.g., from a block in the next month and/or jumping to the end of the measured data. This function may also be achieved by scrolling forward and backward.

In a further embodiment of the invention, the start switches, the retrieval switches and/or buttons/keys and/or the navigation switches and/or rocker switch may be designed in the form of mechanical buttons. Alternatively, other designs of switches and/or buttons may also be provided. For example, the buttons may be implemented by a touch screen or a scroll wheel.

The invention is explained in greater detail below on the basis of a preferred exemplary embodiment and the respective drawings, in which:
- Figure 1: shows a schematic perspective view of blood pressure monitor attached to the upper narrow side of a user's wrist, shown in a sectional diagram according to an advantageous embodiment of the invention,
- Figure 2: shows a schematic diagram of an operating part of a blood pressure monitor from the preceding figure,
- Figure 3: shows a schematic diagram of the operating part of the blood pressure monitor from Figure 1 according to an alternative embodiment of the invention and
- Figure 4: shows a schematic diagram of a navigation switch for navigating within a data set displayed on the device display.

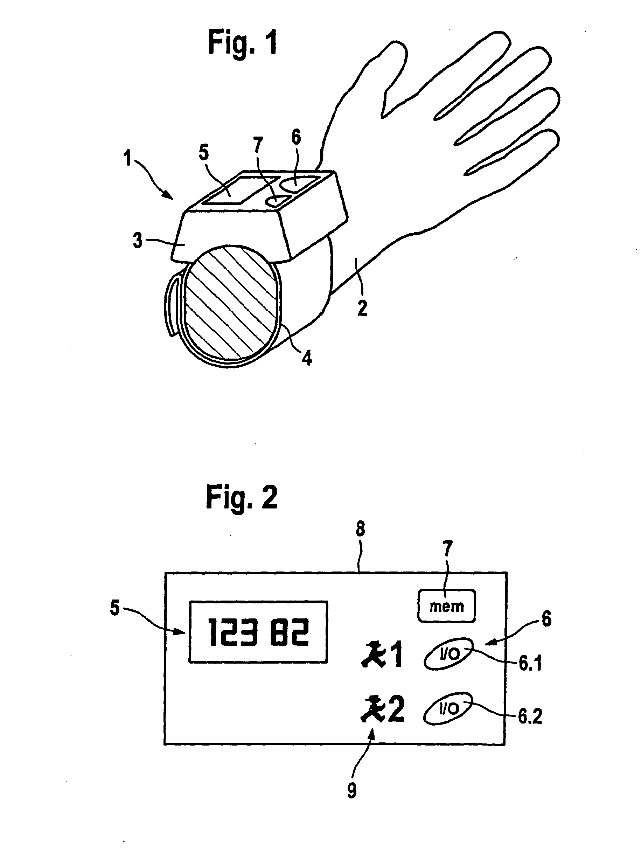

The blood pressure monitor 1 according to Figure 1 comprises a cuff 4 which may applied to the wrist in the embodiment illustrated here or applied to the user's finger or upper arm in variants not shown here. The housing 3 of the blood pressure monitor is preferably attached to the cuff 4, whereby the cuff and the housing may be designed so that the housing 3 is arranged with the display device 5 on the upper narrow side of the wrist 2. In addition to the display device 5 in the form of a display, multiple start/off switches 6 and one memory button 7 are provided on the housing 3. In addition, an electronic control device (not shown here in detail) and a memory (also not shown) having multiple memory traces and/or sites which are able to store the measured values of the individual measurements are also provided in the interior of the housing 3. The blood pressure monitor is equipped with an inclination sensor or a position sensor for detecting the inclination/position of the device in relation to the level of the heart and is equipped with a device for determining the day and time of the measurement and with graphic and arithmetic analytical means (averaging of past measurements at certain intervals or at certain measurement points in time). This "measurement data" of the data accompanying the actual blood pressure measurement may also be stored and retrieved on an individualized basis as described below. Thus, an individualized validity check of the (blood pressure) measurement data is possible, depending on the measurement position during the blood pressure measurement or depending on the time of day of the measurement.

In the embodiment of the operating part 8 of the blood pressure monitor 1 shown in Figure 2, two start/off 6.1 and 6.2 are provided, arranged one above the other in the embodiment shown here. The two start/off switches 6.1 and 6.2 are individualized to be distinguishable from one another by individual optical markings. In the embodiment shown here, the start and off switches 6 are themselves provided with different color markings. In addition, various pictograms 9 symbolizing the various users, namely a user 1 and a user 2 in the embodiment shown here, are also provided directly by the start/off switches 6.

With the operating part 8 shown in Figure 2, the blood pressure monitor 1 is switched on by a first user by depressing the start/off switch 6.1 and a corresponding measurement is initiated. The measured values obtained in this way are automatically assigned to a first memory site and stored there. However, if the devices is started by a second user by depressing the second start/off switch 6.2, then the measured values obtained by the following measurement operation are assigned to a second memory site and stored there. This allows intuitive operation which is further supported by the pictograms 9 next to the start/off switches 6.1 and 6.2 and their color coding.

To display the measured values stored in the memory on the display device 5, the retrieval and/or memory button 7 may be depressed; in the embodiment shown according to Figure 2, this button is arranged above the start/off switches. To individually retrieve and select the measured values of a particular user, in addition to depressing the memory button 7, one of the start/off switches 6.1 and 6.2 may also be depressed, so that only the values of the corresponding memory site are displayed.

The display device 5 is advantageously designed so that a symbol characterizing the respective memory site is displayed together with the measured values being displayed on the display device 5, such that this symbol individualizes the particular set of measured values displayed. A symbol is preferably displayed on the display device 5, corresponding to the optical marking of the respective start/off switch 6.1 or 6.2. In particular, the pictogram 9 characterizing the respective start/off switch 6.1 or 6.2 may be displayed on the display device 5 together with the corresponding measured values.

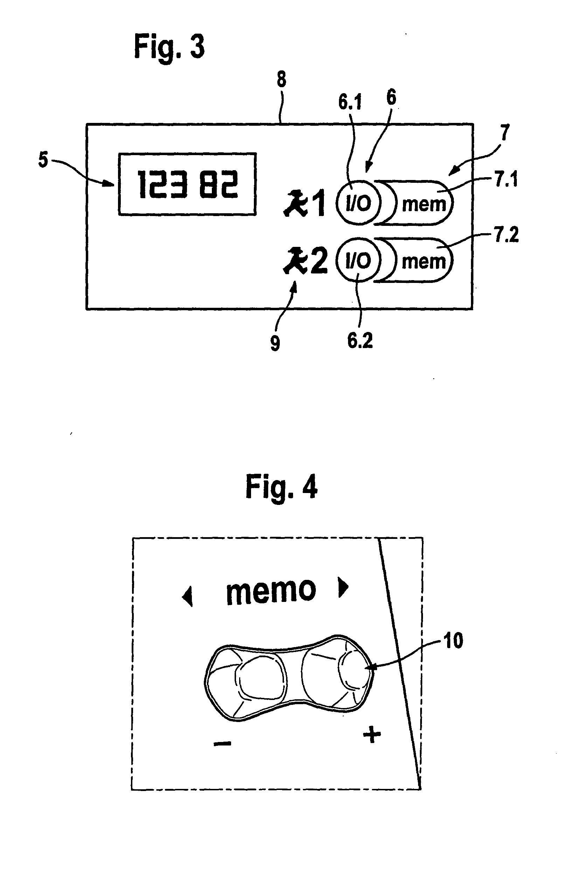

In the alternative embodiment of an operating part 8 shown in Figure 3, two start/off switches 6.1 and 6.2 which are individualized by different pictograms 9 next to the switches are provided here as in the embodiment described previously according to Figure 2. As an alternative to the embodiment according to Figure 2, multiple individualized retrieval buttons 7.1 and 7.2 are provided for retrieving the sets of measured values stored in the memory so that the particular set of measured values desired can be retrieved individually and displayed by the display device 5. In this embodiment, it is not necessary to depress the start/off switch 6 repeatedly to select a certain set of measured values.

The multiple retrieval buttons 7.8 and 7.2 are advantageously arranged in the immediate vicinity of the respective start/off switches 6.1 and 6.2 so that they are individualized by the respective same pictogram 9. This supports the operation of the device in that retrieval of the respective set of stored measured values is possible intuitively by depressing the respective memory button 7.

As Figure 4 shows, in addition to the memory retrieval button 7 and/or the multiple retrieval buttons 7, a navigation switch 10 may also be provided on the operating part 8, facilitating navigation through the particular set of measured values retrieved and displayed. In the embodiment shown here, the navigation switch 10 is designed as a rocker switch which causes the measured values displayed to scroll forward by depressing the button in one direction and causes the measured values displayed to scroll in reverse by depressing the button in the opposite direction. Keeping the button depressed for a longer period of time can cause it to scroll through the values in the memory area.

As an alternative to an application in the blood pressure monitor, the object described above may be also be used in another measurement device for measuring a physiological variable such as the blood sugar level, the body temperature or in a device for processing a certain insulin quantity.

## Claims

1. Blood pressure monitor having a data processing unit for managing measured data, **characterized in that** multiple individualizable start switches (6) are provided for starting the device and/or a measurement operation, wherein the start switches (6) are coupled to the data processing unit in such a way that an individualizable measured data processing and/or management is/are possible by operating a particular start switch (6).

2. Blood pressure monitor according to the preceding claim, wherein the data processing unit is provided with a memory for storing measured data, wherein the memory has multiple memory sites for individualizable storage of various sets of measured data and wherein selection means are provided for selecting a particular memory site for the measured data of a particular measurement.

3. Blood pressure monitor according to the preceding claim, wherein the individualizable start switches (6) are coupled to the selection means in such a way that by operating one of the start switches (6) a respective memory site for the particular measurement is selectable.

4. Blood pressure monitor according to the preceding claim, wherein the start switches (6) and the selection means are designed so that selection of the particular memory site for the particular measurement is performed automatically by starting the particular measurement operation by actuating the particular start button.

5. Blood pressure monitor according to any one of the preceding claim, wherein the operation of one of the start buttons (6) selects the memory site for the particular measurement to follow.

6. Blood pressure monitor according to any one of the preceding claims, wherein after starting the device by operating a start button (6.1), the selected memory site can be switched by operating another start switch (6.2) for the following measurement.

7. Blood pressure monitor according to any one of the preceding claims, wherein the start switches (6) are formed by start buttons (6.1; 6.2) which are individualized by individual-and/or user-specific optical markings or are individualizable by the user.

8. Blood pressure monitor according to the preceding claim, wherein the start buttons (6.1; 6.2) are individualized by various pictograms (9) and/or various color markings.

9. Blood pressure monitor according to any one of the preceding Claims 1 through 6, wherein the start switches (6) are connected to a joint soft start button to which various user identifiers are allocated and which can be individualized, whereby a particular start switch can be triggered as a function of the occupation of the soft start button with a particular user identifier.

10. Blood pressure monitor according to the preceding claim, wherein the soft start button can be connected to a selection menu that shows the various user identifiers and from which a desired user identifier may be selected for allocation to the soft start button.

11. Blood pressure monitor according to Claim 9 or 10, wherein the soft start button can be linked to an input menu by which an individual user identifier can be input.

12. Blood pressure monitor according to any one of the two preceding claims, wherein the data processing unit contains a start lock that activates the soft start button only after a user identifier has been allocated to it.

13. Blood pressure monitor according to any one of the preceding claims, wherein the start switches (6) at the same time form off switches for turning off the device and/or for terminating a measurement operation.

14. Blood pressure monitor according to the preceding claim, wherein a particular measurement operation can be terminated by operating any of the start switches and off switches (6) and/or the device can be turned off by operating any of the start switches and off switches (6).

15. Blood pressure monitor according to any one of the preceding claims, wherein a display device (5) is provided for display of the stored measured values and retrieval means are provided for selecting a particular memory site for display of the respective desired set of measured values to be displayed.

16. Blood pressure monitor according to the preceding claim, wherein multiple individualized retrieval buttons (7.1; 7.2) are provided for retrieving a certain set of measured values each.

17. Blood pressure monitor according to the preceding claim, wherein the retrieval buttons (7) have individualized and/or individualizable optical markings, preferably pictograms or color markings or character fields.

18. Blood pressure monitor according to the preceding claim, wherein the particular start switches (6) and retrieval buttons (7) assigned to a shared memory site are individualized and/or individualizable by corresponding markings.

19. Blood pressure monitor according to Claim 9, wherein the retrieval means have a shared retrieval button (7) for all memory sites, said button being coupled to the individualized start buttons (6) such that by operation of the joint retrieval button (7) and additional operation of one of the start switches (6) the memory site assigned to the particular start switch (6) can be read out and the corresponding measured values can be displayed.

20. Blood pressure monitor according to any one of the preceding claims, wherein individualized sets of measured value symbols may be displayed by the display device (5) together with the measured values, characterizing the particular set of measured values and/or the respective memory site.

21. Blood pressure monitor according to the preceding claim, wherein the individualized measured value symbols correspond to the optical markings on the start buttons (6).
